# EUROPÄISCHE PATENTSCHRIFT

(11) **EP 3 150 258 B1**
(45) Veröffentlichungstag und Bekanntmachung des Hinweises auf die Patenterteilung: **04.09.2019**
(21) Anmeldenummer: 16189163.5
(22) Anmeldetag: 16.09.2016
(51) Int. Cl.: A61Q 17/04, A61K 8/49

(54) **SONNENSCHUTZMITTEL MIT REDUZIERTER TEXTILVERFLECKUNG DURCH BIS-ETHYLHEXYLOXYPHENOL METHOXYPHENYL TRIAZINE**
SUNSCREEN WITH REDUCED TEXTILE STAINING DUE TO BIS-ETHYLHEXYLOXYPHENOL METHOXYPHENYL TRIAZINE
SYSTÈME DE PROTECTION SOLAIRE RÉDUISANT LES TACHES SUR LES TEXTILES PAR LA BIS-ETHYLHEXYLOXYPHENOL METHOXYPHENYL TRIAZINE

(30) Priorität: 01.10.2015 DE 102015219006
(43) Veröffentlichungstag der Anmeldung: 05.04.2017
(73) Patentinhaber: Beiersdorf AG, 20253 Hamburg (DE)
(72) Erfinder: Weinert, Katrin, 22763 Hamburg (DE); Borchers, Kathrin, 21614 Buxtehude (DE); Bleckmann, Andreas, 22926 Ahrensburg (DE)

(56) Entgegenhaltungen:
- WO-A2-2013/000743
- WO-A2-2014/012699
- DE-A1-102010 008 320
- DE-A1-102010 050 774
- DE-A1-102012 212 531
- DE-A1-102013 209 904
- DE-A1-102014 206 156
- US-A1- 2013 309 185
- US-A1- 2014 308 220
- US-A1- 2015 272 848
- DATABASE GNPD [Online] MINTEL; April 2013 (2013-04), "Sun Milk SPF 50+", XP002764351, Database accession no. 2055639
- DATABASE GNPD [Online] MINTEL; April 2015 (2015-04), "Face cream SPF 50", XP002764290, Database accession no. 3101737
- DATABASE GNPD [Online] MINTEL; Juli 2015 (2015-07), "Face cream SPF 50", XP002764291, Database accession no. 3354913

## Beschreibung

Die vorliegende Erfindung betrifft eine kosmetische Zubereitung enthaltend 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) und 1-Hydroxy-4-methyl-6-(2,4,4-trimethylpentyl)-2(1H)-pyridon (Pirocton) oder Piroctone Olamine.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der inhaltsstoffe, insbesondere auf hellen Textilien Flecken und Verfärbungen. Diese Verfärbungen werden insbesondere durch nicht-wasserlösliche UVA-und Breitbandfilter wie 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche Breitbandfilter wie Bis-Ethylhexyloxyphenol Methoxyphenyl Triazine zu entwickeln, welche sich leichter aus den mit der Zubereitung kontaminierten Textilen herauswaschen lassen.

Überraschend gelöst wird die Aufgabe durch eine kosmetische Zubereitung enthaltend 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und
0,25 bis 0,5 Gewichts-%, bezogen auf das Gesamtgewicht der Zubereitung 1-Hydroxy-4-methyl-6-(2,4,4-trimethylpentyl)-2(1H)-pyridon (Pirocton) oder
0,1 bis 0,5 Gewichts-%, bezogen auf das Gesamtgewicht der Zubereitung, Piroctone Olamine.

Zwar kennt der Stand der Technik die DE102004011111, doch konnte diese Schrift nicht den Weg zur vorliegenden Erfindung weisen, weil sie sich mit dem Effekt der Fotostabilisierung von Dibenzoylmethanderivaten befasst. Darüber hinaus kennt der Fachmann die DE 102010054918, DE 10201005465, DE 102010054866, DE 19639817, EP0928193, EP 1088546 und EP0463780, die ebenfalls nicht den Weg zur vorliegenden Erfindung weisen konnten.

Nicht zuletzt kennt der Fachmann die Einträge in der GNPD Datenbank (Mintel) Record ID 2055639 (Sun Milk 50+), Record ID 3101737 (Face Cream SPF 50) und Record ID 3354913 (Face Cream SPF 50), sowie die Offenlegungsschriften DE 10 2013 209904 A1, WO 2014/012699 A2, WO 2013/000743 A2, DE 10 2012 212531 A1, US 2013/309185 A1, DE 10 2014 206156 A1, US 2015/272848 A1, US 2014/308220 A1, DE 10 2010 050774 A1 und DE 10 2010 008320 A1, die ebenfalls nicht den Weg zur vorliegenden Erfindung weisen konnten.

Es ist erfindungsgemäß vorteilhaft, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in einer Menge von 0,01 bis 10,0 Gewichts-%, bezogen auf das Gesamtgewicht der Zubereitung, enthält. Erfindungsgemäß bevorzugt ist dabei ein Gehalt von 1 bis 5 Gewichts-%, bezogen auf das Gesamtgewicht der Zubereitung.

Der Zusatz von Pirocton kann dabei erfindungsgemäß auf zwei Wegen erfolgen. Zum einen kann das Piroton per se der Zubereitung zugesetzt werden. Darüber hinaus kann es aber auch in Form seines Monoethanolamin-Salzes zugesetzt werden. Beide Wege sind erfindungsgemäß.

Die erfindungsgemäß bevorzugte Variante ist jedoch dadurch gekennzeichnet, dass das 1-Hydroxy-4-methyl-6-(2,4,4-trimethylpentyl)-2(1H)-pyridon (Pirocton) in Form seines Monoethanolamin-Salzes (Piroctone Olamine) eingesetzt wird.

Wird das Pirocton in Form von Piroctone Olamine zugefügt, so ist es erfindungsgemäß, diesen Rohstoff in einer Menge von 0,1 bis 0,5 Gewichts-%, bezogen auf das Gesamtgewicht der Zubereitung, einzusetzen.

Erfindungsgemäß vorteilhaft ist es, wenn das Gewichtsverhältnis von 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazin) zu 1-Hydroxy-4-methyl-6-(2,4,4-trimethylpentyl)-2(1H)-pyridon (Pirocton) von 10:1 bis 100:1 beträgt.

Die erfindungsgemäße Zubereitung kann in Form eines alkoholischen Sprays oder Gels oder in Form einer Emulsion vorliegen.

Liegt die Zubereitung in Form eines alkoholischen Sprays oder Gels vor, so ist es erfindungsgemäß vorteilhaft, wenn der Gehalt an Ethanol von 20 bis 70 Gewichts-%, bezogen auf das Gesamtgewicht der Zubereitung beträgt.

Liegt die erfindungsgemäße Zubereitung in Form einer Emulsion vor, so ist es erfindungsgemäß vorteilhaft, wenn es sich dabei um eine O/W-Emulsion handelt.

Darüber hinaus sind derartige Emulsionen als erfindungsgemäß vorteilhaft dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Stearinsäure, Polyglyceryl-10 Stearat, Kaliumcetylphosphat, enthält.

Es ist vorteilhaft im Sinne der vorliegenden Erfindung, wenn die kosmetische Zubereitung dadurch gekennzeichnet ist, dass die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4-(tert.-Butyl)-4'-methoxydibenzoylmethan; 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Erfindungsgemäß bevorzugt ist es dabei, wenn die erfindungsgemäße Zusammensetzung 2-(4'-Diethylamino-2'-hydoxybenzoyl)-benzoesäurehexylester und/oder 4-(tert.-Butyl)-4'-methoxydibenzoylmethan enthält.

Die erfindungsgemäß vorteilhafte Einsatzkonzentration für 4-(tert.-Butyl)-4'-methoxydibenzoylmethan beträgt dabei von 0,01 bis 5 Gewichts-%, bezogen auf das Gesamtgewicht der Zubereitung.

Die erfindungsgemäß vorteilhafte Einsatzkonzentration für 2-(4'-Diethylamino-2'-hydoxybenzoyl)-benzoesäurehexylester beträgt dabei von 0,5 bis 5 Gewichts-%, bezogen auf das Gesamtgewicht der Zubereitung.

Es ist erfindungsgemäß vorteilhaft, wenn die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Darüber hinaus ist es erfindungsgemäß von Vorteil, wenn die Zubereitung Phenoxyethanol und/oder Methylparaben enthält.

Dabei ist es erfindungsgemäß bevorzugt, wenn die Zubereitung Phenoxyethanol in einer Konzentration von 0,01 bis 1 Gewichts-%, bezogen auf das Gesamtgewicht der Zubereitung, enthält.

Es ist erfindungsgemäß von Vorteil, wenn die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

Erfindungsgemäß besonders bevorzugt ist dabei der Einsatz von Glycyrrhetinsäure bzw. Glycyrrhetinsäure enthaltender Pflanzenextrakte (z.B. *Glycyrrhiza glabra*).

Erfindungsgemäß bevorzugt ist es, wenn die Zubereitung frei ist von Propyl- und Butylparaben, 3-Iod-2-propinylbutylcarbamat, 3-(4-Methylbenzyliden)-campher und 2-Hydroxy-4-methoxybenzophenon (Oxybenzon).

Vorteilhaft enthält die erfindungsgemäße Zubereitung Glycerin. Die erfindungsgemäß vorteilhafte Einsatzkonzentration für Glycerin beträgt in den erfindungsgemäßen Zubereitungen von 0,01 bis 15 Gewichts-%, bezogen auf das Gesamtgewicht der Zubereitung.

Erfindungsgemäß vorteilhafte Ausführungsformen erhält man nicht zuletzt dadurch, dass die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

Erfindungsgemäß vorteilhafte Ausführungsformen der vorliegenden Erfindung sind dadurch gekennzeichnet, dass die Zubereitung einen oder mehrere Komplexbildner enthält. Diese können vorteilhaft gewählt werden aus der Gruppe der Verbindungen der EDTA, Aminopolycarboxylate, Polyphosphonate, Polyphosphate und komplexbildenden Säuren.

Dabei ist es erfindungsgemäß bevorzugt, wenn als Komplexbildner eine oder mehrere der Verbindungen aus der Gruppe
- Ethylendiamintetraessigsäure/ EDTA
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
und/oder deren Alkalisalze eingesetzt werden.

Erfindungsgemäß besonders bevorzugte Ausführungsformen der vorliegenden Erfindung sind dadurch gekennzeichnet, dass als Komplexbildner eine oder mehrere der Verbindungen aus der Gruppe
- Ethylendiamintetraessigsäure/ EDTA
- Diethylentriaminpenta(methylenphosphonsäure)/ DTPMP
- Ethylendiamintetra(methylenphosphonsäure/ EDTMP
- Aminotrimethylenphosphonsäure/ ATMP
- Iminodisuccinat
- Natriumpolyphosphat
- Tetranatriumpyrophosphat
- Bernsteinsäure
und/oder deren Alkalisalze eingesetzt werden.

Als erfindungsgemäß vorteilhafte Alkalisalze gelten dabei Natrium- und Kaliumsalze, wobei die Natriumsalze erfindungsgemäß bevorzugt werden.

Es ist vorteilhaft im Sinne der vorliegenden Erfindung, wenn die erfindungsgemäße Zubereitung einen oder mehrere Komplexbildner in einer Gesamtmenge von 0,1 bis 3 Gewichts-%, bezogen auf das Gesamtgewicht der Zubereitung, enthält.

Es ist bevorzugt im Sinne der vorliegenden Erfindung, wenn die erfindungsgemäße Zubereitung einen oder mehrere Komplexbildner in einer Gesamtmenge von 0,5 bis 1,5 Gewichts-%, bezogen auf das Gesamtgewicht der Zubereitung, enthält.

Darüber hinaus ist es vorteilhaft im Sinne der vorliegenden Erfindung, wenn die Zubereitung eine oder mehrere Verbindungen aus der Gruppe der Polysaccharide enthält.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Es ist vorteilhaft im Sinne der vorliegenden Erfindung, wenn die Zubereitung ein oder mehrere Verdickungsmittel enthält.

Erfindungsgemäß bevorzugt werden dabei ein oder mehrere Verdickungsmittel gewählt aus der Gruppe der Verbindungen Ammonium Acryloyldimethyltaurate/VP Copolymer, Ammonium Acryloyldimethyltaurate/ Beheneth-25 Methacrylate Copolymer, Hydroxypropylcellulose Vinylpyrrolidon/Acrylsäure-Copolymer enthält.

Es ist insbesondere von Vorteil, die Filmbildner aus der Gruppe der Polymere auf Basis von Polyvinylpyrrolidon (PVP) 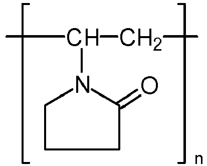 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß ist darüber hinaus ein Verfahren zur Erleichterung der Auswaschbarkeit von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen aus Textilien, dadurch gekennzeichnet, dass dem Kosmetikum 1-Hydroxy-4-methyl-6-(2,4,4-trimethylpentyl)-2(1H)-pyridon (Pirocton) oder dessen Monoethanolamin-Salz zugesetzt wird. Dabei kann in diesem Verfahren das Kosmetikum wie oben beschrieben zusammengesetzt sein.

Erfindungsgemäß ist nicht zuletzt die Verwendung von 1-Hydroxy-4-methyl-6-(2,4,4-trimethylpentyl)-2(1H)-pyridon (Pirocton) und/oder dessen Monoethanolamin-Salz in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen nach einem der vorhergehenden Ansprüche zur Erleichterung der Auswaschbarkeit der UV-Lichtschutzfilter aus mit den Zubereitungen kontaminierten Textilien. Dabei kann auch hier das Kosmetikum wie oben beschrieben zusammengesetzt sein.

### Vergleichsversuch

Mit dem folgenden Versuch konnte der erfindungsgemäße Effekt beispielhaft belegt werden: Es wurden jeweils 0,5% des erfindungsgemäßen Hilfsstoffs Pirocton bzw. dessen Monoethanolamin-Salzes zu einer Bis-Ethylhexyloxyphenol Methoxyphenyl Triazin enthaltenden Formulierung zugesetzt und die verfleckungsreduzierende Wirkung (Reduktion db) im Vergleich zu einer Formulierung ohne den erfindungsgemäßen Hilfsstoff mittels einer *in vitro* Waschmethode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 dargestellt sind.

Es wurden verschiedene Sonnenschutzemulsionen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, 10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; db-Wert [%]**

| **INCI** | **Beispiel [%]** | | |
|---|---|---|---|
| | Rezeptur 1 | Rezeptur 2 | Rezeptur 3 |
| Piroctone | | | 0,50 |
| Piroctone Olamine | | 0,50 | |
| Bis-Ethylhexyloxyphenol Methoxyphenyl Triazine | 3,50 | 3,50 | 3,50 |
| Myristyl Myristate | 1,00 | 1,00 | 1,00 |
| C18-38 Alkyl Hydroxystearoyl Stearate | 0,50 | 0,50 | 0,50 |
| Glyceryl Stearate | 1,00 | 1,00 | 1,00 |
| Sodium Cetearyl Sulfate | 0,15 | 0,15 | 0,15 |
| Silica Dimethyl Silylate | 0,50 | 0,50 | 0,50 |
| VP/Hexadecene Copolymer | 0,50 | 0,50 | 0,50 |
| Glycerin | 8,00 | 8,00 | 8,00 |
| Phenoxyethanol | 0,50 | 0,50 | 0,50 |
| Ethylparaben | 0,10 | 0,10 | 0,10 |
| Methylparaben | 0,30 | 0,30 | 0,30 |
| Cetearyl Alcohol | 1,00 | 1,00 | 1,00 |
| Acrylates/C10-30 Alkyl Acrylate Crosspolymer | 0,05 | 0,05 | 0,05 |
| Xanthan Gum | 0,40 | 0,40 | 0,40 |
| Alcohol Denat. | 4,00 | 4,00 | 4,00 |
| Trisodium EDTA | 0,20 | 0,20 | 0,20 |
| Homosalate | 9,50 | 9,50 | 9,50 |
| Ethylhexyl Salicylate | 4,75 | 4,75 | 4,75 |
| Butyl Methoxydibenzoylmethane | 5,00 | 5,00 | 5,00 |
| Titanium Dioxide | 3,50 | 3,50 | 3,50 |
| Phenylbenzimidazole Sulfonic Acid | 1,00 | 1,00 | 1,00 |
| Octocrylene | 9,50 | 9,50 | 9,50 |
| Parfum | q.s. | q.s. | q.s. |
| Sodium Hydroxide | q.s. | q.s. | q.s. |
| Aqua | ad 100 | ad 100 | ad 100 |
| | | | |
| Gelbwert-Reduktion db [%] | - | -35 | -28 |

**Fazit:** Die Auswaschbarkeit der durch Bis-Ethylhexyloxyphenol Methoxyphenyl Triazin hervorgerufenen Textilverfärbung wird durch den Zusatz von Piroctone bzw. Piroctone Olamine im Sonnenschutzmittel verbessert.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **Beispiel [%]** | | | | |
|---|---|---|---|---|---|
| | Rezeptur 4 | Rezeptur 5 | Rezeptur 6 | Rezeptur 7 | Rezeptur 8 |
| Bis-Ethylhexyloxyphenol Methoxyphenyl Triazine | 3,50 | 3,50 | 4,00 | 4,00 | 3,50 |
| Triacontanyl PVP | | | | | 1,00 |
| Piroctone | 0,50 | | | 0,50 | |
| Piroctone Olamine | | 0,45 | 0,45 | | 0,45 |
| Myristyl Myristate | 1,00 | 1,00 | 0,00 | | |
| C18-38 Alkyl Hydroxystearoyl Stearate | 0,50 | 0,50 | 1,00 | | |
| Dibutyl Adipate | | | 3,00 | | |
| C12-15 Alkyl Benzoate | | | 5,00 | | |
| Butylene Glycol Dicaprylate/Dicaprate | | | 4,00 | 3,00 | 2,00 |
| C18-36 Acid Triglyceride | | | | 0,50 | 1,00 |
| Glyceryl Stearate SE | 1,00 | 1,00 | | | |
| Sodium Cetearyl Sulfate | 0,15 | 0,15 | | | |
| Glyceryl Stearate | | | 1,00 | | |
| Sodium Stearoyl Glutamate | | | 0,30 | | |
| Ceteareth-20 | | | | 1,00 | 1,50 |
| Silica Dimethyl Silylate | 0,50 | 0,50 | 1,00 | | |
| VP/Hexadecene Copolymer | 0,50 | 0,50 | 0,50 | 1,00 | |
| Perfume | q.s. | q.s. | q.s. | q.s. | q.s. |
| Glycerin | 8,00 | 8,00 | 7,50 | 5,00 | 5,00 |
| Sodium Hydroxide | q.s. | q.s. | q.s. | q.s. | q.s. |
| Phenoxyethanol | 0,50 | 0,50 | 0,50 | 0,50 | 0,50 |
| Ethylparaben | 0,10 | | | 0,20 | 0,20 |
| Methylparaben | 0,30 | 0,30 | | 0,30 | 0,30 |
| Cetearyl Alcohol | 1,00 | 1,00 | | | |
| Acrylates/C10-30 Alkyl Acrylate Crosspolymer | 0,05 | 0,10 | 0,10 | 0,25 | 0,35 |
| Xanthan Gum | 0,40 | 0,40 | 0,40 | | |
| Stearyl Alcohol | | | 1,00 | | |
| Alcohol Denat. | 4,00 | 4,00 | 3,00 | 4,00 | 4,00 |
| Trisodium EDTA | 0,20 | 0,20 | 0,20 | 0,20 | 0,20 |
| Homosalate | 9,50 | 9,50 | 0,00 | 9,00 | 9,00 |
| Ethylhexyl Salicylate | 4,75 | 4,75 | 4,75 | 4,50 | 4,75 |
| Butyl Methoxydibenzoylmethane | 4,75 | 4,75 | 4,75 | 4,50 | 4,50 |
| Octocrylene | 9,50 | 9,50 | | 8,00 | 8,00 |
| Titanium Dioxide + Silica + Dimethicone | 3,50 | | | | |
| Phenylbenzimidazole Sulfonic Acid | 1,00 | 1,00 | 1,00 | 1,50 | 1,50 |
| Ethylhexyl Triazone | | | 3,00 | | |
| Aqua | ad 100 | ad 100 | ad 100 | ad 100 | ad 100 |

## Patentansprüche

1. Kosmetische Zubereitung enthaltend
2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und
0,25 bis 0,5 Gewichts-%, bezogen auf das Gesamtgewicht der Zubereitung 1-Hydroxy-4-methyl-6-(2,4,4-trimethylpentyl)-2(1H)-pyridon (Pirocton) oder
0,1 bis 0,5 Gewichts-%, bezogen auf das Gesamtgewicht der Zubereitung, Piroctone Olamine.

2. Kosmetische Zubereitung nach Anspruch 1, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in einer Menge von 0,01 bis 10,0 Gewichts-%, bezogen auf das Gesamtgewicht der Zubereitung, enthält.

3. Kosmetische Zubereitung nach einem der vorhergehenden Ansprüche, **dadurch gekennzeichnet, dass** das1-Hydroxy-4-methyl-6-(2,4,4-trimethylpentyl)-2(1H)-pyridon (Pirocton) in Form seines Monoethanolamin-Salzes (Piroctone Olamine) eingesetzt wird.

4. Kosmetische Zubereitung nach einem der vorhergehenden Ansprüche, **dadurch gekennzeichnet, dass** das Gewichtsverhältnis von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), zu 1-Hydroxy-4-methyl-6-(2,4,4-trimethylpentyl)-2(1H)-pyridon (Pirocton) von 10:1 bis 100:1 beträgt.

5. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Stearinsäure, Polyglyceryl-10 Stearat, Kaliumcetylphosphat, enthält.

6. Kosmetische Zubereitung nach einem der vorhergehenden Ansprüche, **dadurch gekennzeichnet, dass** die Zubereitung einen oder mehrere UV-Filterenthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sultonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4-(tert.-Butyl)-4'-methoxydibenzoylmethan; 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

7. Kosmetische Zubereitung nach einem der vorhergehenden Ansprüche, **dadurch gekennzeichnet, dass** die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

8. Kosmetische Zubereitung nach einem der vorhergehenden Ansprüche, **dadurch gekennzeichnet, dass** die Zubereitung Phenoxyethanol und/oder Methylparaben enthält.

9. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

10. Kosmetische Zubereitung nach einem der vorhergehenden Ansprüche, **dadurch gekennzeichnet, dass** die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

11. Kosmetische Zubereitung nach einem der vorhergehenden Ansprüche, **dadurch gekennzeichnet, dass** die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

12. Kosmetische Zubereitung nach einem der vorhergehenden Ansprüche, **dadurch gekennzeichnet, dass** die Zubereitung einen oder mehrere Komplexbildner enthält, gewählt aus der Gruppe der Verbindungen der EDTA, Aminopolycarboxylate, Polyphosphonate, Polyphosphate und komplexbildenden Säuren.

13. Kosmetische Zubereitung nach einem der vorhergehenden Ansprüche, **dadurch gekennzeichnet, dass** die Zubereitung eine oder mehrere Verbindungen aus der Gruppe der Polysaccharide enthält.

14. Verwendung von 1-Hydroxy-4-methyl-6-(2,4,4-trimethylpentyl)-2(1H)-pyridon (Pirocton) und/oder dessen Monoethanolamin-Salz in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen nach einem der vorhergehenden Ansprüche 1 bis 13 zur Erleichterung der Auswaschbarkeit der UV-Lichtschutzfilter aus mit den Zubereitungen kontaminierten Textilien.

## Claims

1. Cosmetic preparation comprising 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and from 0.25 to 0.5% by weight, based on the total weight of the preparation, of 1-hydroxy-4-methyl-6-(2,4,4-trimethylpentyl)-2(1H)-pyridone (piroctone) or from 0.1 to 0.5% by weight, based on the total weight of the preparation, of piroctone olamine.

2. Cosmetic preparation according to Claim 1, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) in an amount of from 0.01 to 10.0% by weight, based on the total weight of the preparation.

3. Cosmetic preparation according to one of the preceding claims, **characterized in that** the 1-hydroxy-4-methyl-6-(2,4,4-trimethylpentyl)-2(1H)-pyridone (piroctone) is used in the form of its monoethanolamine salt (piroctone olamine).

4. Cosmetic preparation according to one of the preceding claims, **characterized in that** the weight ratio of 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) to 1-hydroxy-4-methyl-6-(2,4,4-trimethylpentyl)-2(1H)-pyridone (piroctone) is from 10:1 to 100:1.

5. Cosmetic preparation according to one of the preceding claims, **characterized in that** the preparation comprises one or more emulsifiers selected from the group of the compounds glyceryl stearate citrate, cetearyl alcohol, sodium cetearyl sulphate, glyceryl stearate, cetearyl sulphosuccinate, sodium stearoylglutamate, polyglyceryl-3 methylglucose distearate, stearic acid, polyglyceryl-10 stearate, potassium cetyl phosphate.

6. Cosmetic preparation according to one of the preceding claims, **characterized in that** the preparation comprises one or more UV filters which are selected from the group of the compounds 2-phenylbenzimidazole-5-sulphonic acid and/or salts thereof; phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethyl benzalmalonate; 3-(4-(2,2-bisethoxycarbonylvinyl)phenoxy)propenyl)methoxysiloxane/dimethylsiloxane copolymer; hexyl 2-(4'-diethylamino-2'-hydoxybenzoyl)benzoate; dioctylbutylamidotriazone (INCI: Diethylhexyl-Butamidotriazone); 2,4-bis[5-1(dimethylpropyl)benzoxazol-2-yl-(4-phenyl)imino]-6-(2-ethylhexyl)imino-1,3,5-triazine with the CAS No. 288254-16-0; 4-(tert-butyl)-4'-methoxydibenzoylmethane; tris(2-ethylhexyl) 4,4',4"-(1,3,5-triazine-2,4,6-triyltriimino)trisbenzoate (also: 2,4,6-tris-[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanines; titanium dioxide; zinc oxide.

7. Cosmetic preparation according to one of the preceding claims, **characterized in that** the preparation comprises one or more compounds selected from the group of the compounds ethylhexylglycerol, polyglyceryl-2 caprate, propylene glycol, butylene glycol, 2-methylpropane-1,3-diol, 1,2-pentanediol, 1,2-hexanediol, 1,2-octanediol and/or 1,2-decanediol.

8. Cosmetic preparation according to one of the preceding claims, **characterized in that** the preparation comprises phenoxyethanol and/or methylparaben.

9. Cosmetic preparation according to one of the preceding claims, **characterized in that** the preparation comprises one or more active ingredients selected from the group of the compounds magnolia extract, alpha-lipoic acid, folic acid, phytoene, D-biotin, coenzyme Q10, alpha-glucosylrutin, carnitine, carnosine, polydocanol, natural and/or synthetic isoflavonoids, flavonoids, creatine, creatinine, taurine, β-alanine, tocopheryl acetate, panthenol, magnolol, honokiol, urea, hyaluronic acid, dihydroxyacetone, 8-hexadecene-1,16-dicarboxylic acid, glycyrrhetic acid, glucosyl glycerides and/or licochalcone A.

10. Cosmetic preparation according to one of the preceding claims, **characterized in that** the preparation is free from propyl- and butylparaben, 3-iodo-2-propynyl butylcarbamate, 3-(4-methylbenzylidene)camphor and 2-hydroxy-4-methoxybenzophenone (oxybenzone).

11. Cosmetic preparation according to one of the preceding claims, **characterized in that** the preparation comprises C12-15 alkyl benzoate, cyclomethicone, octyldodecanol, caprylic/capric acid triglycerides, butylene glycol dicaprylate/dicaprate, phenethyl benzoate, cocoglycerides, di-n-butyl adipate and/or diisopropyl adipate.

12. Cosmetic preparation according to one of the preceding claims, **characterized in that** the preparation comprises one or more complexing agents selected from the group of the compounds of the EDTAs, aminopolycarboxylates, polyphosphonates, polyphosphates and complexing acids.

13. Cosmetic preparation according to one of the preceding claims, **characterized in that** the preparation comprises one or more compounds from the group of polysaccharides.

14. Use of 1-hydroxy-4-methyl-6-(2,4,4-trimethylpentyl)-2(1H)-pyridone (piroctone) and/or the monoethanolamine salt thereof in cosmetic preparations comprising 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) according to one of the preceding Claims 1 to 13 for facilitating the ability of the UV light protection filters to be washed out of textiles contaminated with the preparations.

## Revendications

1. Préparation cosmétique contenant :
de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) et
0,25 à 0,5 % en poids, par rapport au poids total de la préparation, de 1-hydroxy-4-méthyl-6-(2,4,4-triméthylpentyl)-2(1H)-pyridone (piroctone) ou
0,1 à 0,5 % en poids, par rapport au poids total de la préparation, de piroctone olamine.

2. Préparation cosmétique selon la revendication 1, **caractérisée en ce que** la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) en une quantité de 0,01 à 10,0 % en poids, par rapport au poids total de la préparation.

3. Préparation cosmétique selon l'une quelconque des revendications précédentes, **caractérisée en ce que** la 1-hydroxy-4-méthyl-6-(2,4,4-triméthylpentyl)-2(1H)-pyridone (piroctone) est utilisée sous la forme de son sel de monoéthanolamine (piroctone olamine).

4. Préparation cosmétique selon l'une quelconque des revendications précédentes, **caractérisée en ce que** le rapport en poids entre la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) et la 1-hydroxy-4-méthyl-6-(2,4,4-triméthylpentyl)-2(1H)-pyridone (piroctone) est de 10:1 à 100:1.

5. Préparation cosmétique selon l'une quelconque des revendications précédentes, **caractérisée en ce que** la préparation contient un ou plusieurs émulsifiants choisis dans le groupe de composés constitué par le stéarate-citrate de glycéryle, l'alcool cétéarylique, le sulfate de cétéaryle sodium, le stéarate de glycéryle, le sulfosuccinate de cétéaryle, le glutamate de stéaroyle sodium, le distéarate de polyglycéryl-3-méthylglucose, l'acide stéarique, le stéarate de polyglycéryle-10, le phosphate de cétyle potassium.

6. Préparation cosmétique selon l'une quelconque des revendications précédentes, **caractérisée en ce que** la préparation contient un ou plusieurs filtres UV, qui sont choisis dans le groupe de composés constitué par l'acide 2-phénylbenzimidazole-5-sulfonique et/ou ses sels, les sels de l'acide phénylène-1,4-bis-(2-benzimidazyl)-3,3'-5,5'-tétrasulfonique, le 1,4-di(2-oxo-10-sulfo-3-bornylidène-méthyl)-benzène et ses sels, les sels de l'acide 4-(2-oxo-3-bornylidène-méthyl)benzène-sulfonique, les sels de l'acide 2-méthyl-5-(2-oxo-3-bornylidène-méthyl)sulfonique, le 2,2'-méthylène-bis-(6-(2H-benzotriazol-2-yl)-4-(1,1,3,3-tétraméthylbutyl)-phénol), le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol, le 3-(4-méthylbenzylidène)camphre, le 3-benzylidène-camphre, le salicylate d'éthylhexyle, l'acide téréphtalidène-dicamphre-sulfonique, l'acrylate de 2-éthylhexyl-2-cyano-3,3-diphényle, l'ester 2-éthylhexylique de l'acide 4-(diméthylamino)-benzoïque, l'ester amylique de l'acide 4-(diméthylamino)benzoïque, l'ester di(2-éthylhexylique) de l'acide 4-méthoxybenzalmalonique, l'ester 2-éthylhexylique de l'acide 4-méthoxycinnamique, l'ester isoamylique de l'acide 4-méthoxycinnamique, la 2-hydroxy-4-méthoxybenzophénone, la 2-hydroxy-4-méthoxy-4'-méthylbenzophénone la 2,2'-dihydroxy-4-méthoxybenzophénone, le salicylate d'homomenthyle, le 2-hydroxybenzoate de 2-éthylhexyle, le benzalmalonate de diméthicodiéthyle, le copolymère de 3-(4-(2,2-bis-éthoxycarbonylvinyl)-phénoxy)propényl)-méthoxysiloxane/diméthylsiloxane, l'ester hexylique de l'acide 2-(4'-diéthylamino-2'-hydroxybenzoyl)-benzoïque, la dioctylbutylamidotriazone (INCI: Diethylhexyl-Butamidotriazone), la 2,4-bis-[5-1-(diméthylpropyl)benzoxazol-2-yl-(4-phényl)-imino]-6-(2-éthylhexyl)-imino-1,3,5-triazine de n° CAS 288254-16-0, le 4-(tert.-butyl)-4'-méthoxydibenzoylméthane ; l'ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI: Ethylhexyl Triazone), la 2,4,6-tribiphényl-4-yl-1,3,5-triazine, la mérocyanine, le dioxyde de titane, l'oxyde de zinc.

7. Préparation cosmétique selon l'une quelconque des revendications précédentes, **caractérisée en ce que** la préparation contient un ou plusieurs composés choisis dans le groupe de composés constitué par l'éthylhexylglycérine, le caprate de polyglycéryle-2, le propylène glycol, le butylène glycol, le 2-méthylpropane-1,3-diol, le 1,2-pentanediol, le 1,2-hexanediol, le 1,2-octanediol et/ou le 1,2-décanediol.

8. Préparation cosmétique selon l'une quelconque des revendications précédentes, **caractérisée en ce que** la préparation contient du phénoxyéthanol et/ou du méthylparabène.

9. Préparation cosmétique selon l'une quelconque des revendications précédentes, **caractérisée en ce que** la préparation contient un ou plusieurs agents actifs choisis dans le groupe de composés constitué par l'extrait de magnolia, l'acide alpha-liponique, l'acide folique, le phytoène, la D-biotine, la coenzyme Q10, l'alpha-glucosylrutine, la carnitine, la carnosine, le polydocanol, les isoflavonoïdes naturels et/ou synthétiques, les flavonoïdes, la créatine, la créatinine, la taurine, la β-alanine, l'acétate de tocophéryle, le panthénol, le magnolol, l'honokiol, l'urée, l'acide hyaluronique, la dihydroxyacétone, l'acide 8-hexadécène-1,16-dicarboxylique, l'acide glycyrrhizique, les glucosylglycérides et/ou la licochalcone A.

10. Préparation cosmétique selon l'une quelconque des revendications précédentes, **caractérisée en ce que** la préparation est exempte de propyl- et butylparabène, de carbamate de 3-iodo-2-propinylbutyle, de 3-(4-méthylbenzylidène)-camphre et de 2-hydroxy-4-méthoxybenzophénone (oxybenzone).

11. Préparation cosmétique selon l'une quelconque des revendications précédentes, **caractérisée en ce que** la préparation contient du benzoate d'alkyle en C12-15, de la cyclométhicone, de l'octyldodécanol, du triglycéride caprylique/caprique, du dicaprylate/dicaprate de butylène glycol, du benzoate de phénéthyle, des glycérides de coco, de l'adipate de di-n-butyle et/ou de l'adipate de diisopropyle.

12. Préparation cosmétique selon l'une quelconque des revendications précédentes, **caractérisée en ce que** la préparation contient un ou plusieurs complexants, choisis dans le groupe de composés constitué par l'EDTA, les aminopolycarboxylates, les polyphosphonates, les polyphosphates et les acides complexants.

13. Préparation cosmétique selon l'une quelconque des revendications précédentes, **caractérisée en ce que** la préparation contient un ou plusieurs composés du groupe des polysaccharides.

14. Utilisation de 1-hydroxy-4-méthyl-6-(2,4,4-triméthylpentyl)-2(1H)-pyridone (piroctone) et/ou son sel de monoéthanolamine dans des préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) selon l'une quelconque des revendications 1 à 13 précédentes pour faciliter la rinçabilité des filtres de protection contre la lumière UV à partir de textiles contaminés avec les préparations.
